Europäisches Patentamt

European Patent Office

Office européen des brevets

(11) Veröffentlichungsnummer: **0 297 630**
**A1**

## (12) EUROPÄISCHE PATENTANMELDUNG

(21) Anmeldenummer: 88200123.3

(22) Anmeldetag: 16.01.88

(51) Int. Cl.⁴: **A61K 31/70** , //(A61K31/70, 31:445)

(30) Priorität: 20.01.87 DE 3701497

(43) Veröffentlichungstag der Anmeldung:
04.01.89 Patentblatt 89/01

(84) Benannte Vertragsstaaten:
ES GR

(71) Anmelder: **Frankhof, Wolfgang**
**Meybuschhof 40**
**D-4300 Essen-Katernberg(DE)**

Anmelder: **Thiemer, Klaus**
**Meybuschhof 40**
**D-4300 Essen-Katernberg(DE)**

(72) Erfinder: **Frankhof, Wolfgang**
**Meybuschhof 40**
**D-4300 Essen-Katernberg(DE)**
Erfinder: **Thiemer, Klaus**
**Meybuschhof 40**
**D-4300 Essen-Katernberg(DE)**

(74) Vertreter: **von Kreisler, Alek, Dipl.-Chem. et al**
**Deichmannhaus am Hauptbahnhof**
**D-5000 Köln .1(DE)**

(54) Arzneimittel, enthaltend Lokalanästhetika und Nukleoside sowie dessen Verwendung zur Behandlung von Muskelverletzungen.

(57) Die Erfindung betrifft Arzneimittel, die in wässriger Phase 1 bis 100 g/l einer Lokalanästhetikums, 1 bis 100 g/l eines oder mehrerer Nukleoside aus der Gruppe Adenosin, Guanosin, Inosin, Uridin und deren wasserlöslicher Mono-, Di- und Triphosphatsalze und ein oder mehrere mit den Wirkstoffen verträgliche, physiologisch unbedenkliche Konservierungsmittel sowie gegebenenfalls übliche Träger- und Hilfsstoffe enthalten, sowie die Verwendung derartiger Arzneimittel zur lokalen Infiltrationsbehandlung von Muskelverletzungen.

EP 0 297 630 A1

## Arzneimittel, enthaltend Lokalanästhetika und Nukleoside sowie dessen Verwendung zur Behandlung von Muskelverletzungen

Die Erfindung betrifft ein Arzneimittel, das Lokalanästhetika und ein oder mehrere Nukleoside bzw. deren Phosphorsäureester enthält. Die Erfindung betrifft außerdem die Verwendung derartiger Arzneimittel zur Behandlung von Muskelverletzungen.

Die Zahl der Sporttreibenden hat in jüngster Zeit sowohl im Spitzensport als auch im Breitensport erheblich zugenommen. Dieser Zunahme folgte auch eine deutliche Zunahme der Zahl und Schwere der Sportverletzungen infolge erhöhten Trainingsumfangs, stärkerer Wettkampftätigkeit, höherer Belastung und veränderter Altersstruktur der Sportler. Da sportärztliche Voruntersuchungen nicht oder nur unzureichend durchgeführt werden, nehmen gerade bei Freizeitsportlern die Schäden im Bereich der Muskulatur zu, die durch unkontrollierte und zu hohe Belastung im Sport bedingt sind. Muskelzerrungen, Muskelfaserrisse, Muskelrisse sowie Traumen in Form von Muskelkontusionen und intramuskulären Hämatomen sind die häufigsten Sportverletzungen. Derartige Verletzungen der Muskulatur führen meist unmittelbar zu Leistungsverlust, mehr oder weniger langer Sportunfähigkeit und auch Arbeitsunfähigkeit.

Nach dem aktuellen Kenntnisstand sind derartige Verletzungen am besten dadurch zu heilen, daß die betroffene Muskelpartie über längere Zeit ruhiggestellt oder zumindest nur schwach belastet wird. Dies ist mit einer längeren Leistungspanse oder sogar mit Arbeitsunfähigkeit verbunden.

Im Heilprozeß werden bisher lediglich medicophysikalische und balneologische Maßnahmen ergriffen. Es finden Salbenverbände, Eis- und Fango-Packungen, verschiedene Elektrotherapien sowie Massagen und krankengymnastischer Therapie Anwendung. Außerdem wird versucht, durch Injektionen und Infiltration sowie durch orale Verabreichung verschiedener Medikamente die Phase der Heilung abzukürzen. Vielfach verwendet wurden Antirheumatika, Antiphlogistika, Corticosteroide sowie Medikamente, die in 4-Stellung substituierte Benzoesäure-Derivate, wie p-Hydroxybenzoesäureester oder das Hydrochlorid des p-Aminobenzoesäure-2-(diethylamino-)ethylesters (Procain-Hydrochlorid) enthalten.

Die bisher aufgezählten Maßnahmen entsprechen dem derzeitigen Stand der Therapie, wobei eine signifikante Verkürzung der Krankheitsdauer im Vergleich zu unbehandelten Muskelverletzungen leider nur in geringem Umfang beobachtet werden kann. Die oben beschriebenen Maßnahmen zur Behandlung von Muskelverletzungen führen nicht zu einer wesentlichen Funktionsverbesserung im Heilungsprozeß, sondern beeinflussen lediglich die Schmerzzustände geringfügig und sind zum Teil mit er heblichen Nebenwirkungen behaftet. Diese bestehen darin, daß sich Narben nur schlecht oder mit großer Verzögerung bilden und eine merkliche Rezidivgefahr besteht. Zudem sind zahlreiche der genannten Medikamente nur schlecht magenverträglich oder führen zu Blutbildveränderungen. Darüber wird in folgenden Druckschriften ausführlicher berichtet:

1. H. Hess, "Sportverletzungen" (Herausgeber: Luitpold-Werk);

2. V. Kresci und P. Koch, "Muskelverletzungen und Tendopathie der Sportler", Thieme Verlag, Stuttgart; und

3. H. de Marees "Sportphysiologie".

Die geschilderten therapeutischen Unzulänglichkeiten in der Behandlung von Muskelverletzungen und Muskeltraumen bedingen ein Bedürfnis nach einem schnell wirksamen, nebenwirkungsfreien und universell bei sämtlichen Muskelverletzungen einsetzbaren Medikament. Es wurde überraschend gefunden, daß wässrige Arzneimittel, die bestimmte Lokalanästhetika und ein oder mehrere Nukleoside zusammen mit üblichen Träger- und Hilfsstoffen enthalten, allen Anforderungen bezüglich universeller Anwendbarkeit, Freiheit von Nebenwirkungen und Rezidivfreiheit entsprechen und trotzdem hoch wirksam sind.

Die Erfindung betrifft Arzneimittel, enthaltend in wässriger Phase 1 bis 100 g/l eines Lokalanästhetikums, 1 bis 100 g/l eines oder mehrerer Nukleoside aus der Gruppe Adenosin, Guanosin, Inosin, Uridin und deren wasserlöslicher Mono-, Di- und Triphosphatester-Salze, und ein oder mehrere mit den Wirkstoffen verträgliche, physiologisch unbedenkliche Konservierungsmittel sowie gegebenenfalls übliche Träger- und Hilfsstoffe.

Die Erfindung betrifft außerdem die Verwendung derartiger Arzneimittel zur lokalen Infiltrationsbehandlung von Muskelverletzungen.

Für die erfindungsgemäßen Arzneimittel sind eine Vielzahl von Lokalanästhetika geeignet, die intern wirksam sind. Insbesondere sind dies Lokalanästhetika aus der Gruppe der basischen Säureanilide sowie deren HCl-Addukte, wie beispielsweise 1-Methylpiperidin-2-carbonsäure-N-(2´.6´-dimethylphenyl-)amid (Mepivacain) bzw. dessen Hydrochlorid. Stattdessen oder zusammen damit sind jedoch auch andere Lokalanästhetika geeignet, wenn sie über Injektionen verabreicht werden können.

Das genannte oder mit dem genannten verwandte Lokalanästhetika werden in Konzentrationen von 1 bis 100 g/l wässriger Phase, bevorzugt 10 bis 30 g/l wässriger Phase in dem erfindungsgemäßen Arzneimittel eingesetzt. Besonders bevorzugt werden dafür 0,5- bis 2 %ige Lösungen des jeweiligen Lokalanästhetikums in Mengen zwischen 1 und 20 ml pro Dosis des verabreichten Arzneimittels, bevorzugt in Mengen von 5 bis 10 ml pro Dosis des Arzneimittels, eingesetzt. Die genannten Lösungen, beispielsweise 1 %ige Lösungen, derartiger Lokalanästhetika sind größtenteils kommerziell erhältlich, beispielsweise unter den Namen Meaverin® oder Scandicain®.

Als weitere Komponenten enthalten die erfindungsgemäßen Arzneimittel eines oder mehrere Nukleoside aus der Gruppe Adenosin, Guanosin, Inosin, Uridin und deren wasserlösliche Mono-, Di- und Triphosphatestersalze. Zwar können in den Arzneimitteln einzelne der genannten Nukleoside enthalten sein. Bevorzugt ist jedoch eine Mischung sowohl aller vier genannten Nukleoside als auch eines oder mehrerer ihrer Phosphatsalze.

Bevorzugt werden dabei Natriumsalze, saure Natriumsalze sowie die jeweiligen Nukleosid-Monophosphorsäuren, -diphosphorsäuren und -triphosphorsäuren verwendet. In einer besonders bevorzugten Ausführungsform des erfindungsgemäßen Arzneimittels werden Adenosin, Guanosin, Inosin und Uridin sowie Dinatriumdihydrogenadenosintriphosphat, Adenosindiphosphorsäure, Adenosinmonophosphorsäure und Guanosinmonophosphorsäure verwendet.

Die Mischungsverhältnisse der einzelnen Nukleoside bzw. ihrer Phosphatsalze können dabei in weiten Grenzen schwanken. Bevorzugt werden Adenosin, Guanosin, Inosin und Uridin im Gewichtsverhältnis 5 : 1 : 5 : 1 sowie Dinatriumdihydrogenadenosintriphosphat, Adenosindiphosphorsäure, Adenosinmonophosphorsäure und Guanosinmonophosphorsäure im Gewichtsverhältnis 3 : 1 : 1 : 2 verwendet. Es sind jedoch auch Mischungsverhältnisse einsetzbar, die wesentlich unter oder über den angegebenen Verhältnissen liegen. Die Nukleoside bzw. ihre Gemische können als solche gemischt und dabei oder danach in physiologisch akzeptablen Medien wie Wasser oder physiologischer Kochsalzlösung gelöst werden. Bevorzugt werden jedoch kommerziell erhältliche Mischungen der Nukleoside bzw. ihrer Salze in den genannten Medien, beispielsweise Laevadosin® 2. Die Gesamtkonzentration an den genannten Nukleosidkomponenten in den erfindungsgemäßen Arzneimitteln liegt dabei im Bereich von 1 bis 100 g/l wässriger Phase, bevorzugt im Bereich von 10 bis 30 g/l wässriger Phase.

Zur Konservierung der wässrigen Lösung enthält das erfindungsgemäße Arzneimittel außerdem ein oder mehrere Konservierungsmittel, die mit den oben genannten Wirkstoffen verträglich sein müssen. Als solche sind in erster Linie Chlorkresol und verwandte Verbindungen zu nennen. Diese sind beispielsweise in einer Menge von 0,1 bis 1000 mg/l in den erfindungsgemäßen Arzneimitteln vorhanden.

Außerdem können die erfindungsgemäßen Arzneimittel noch weitere, für derartige Mittel übliche Träger- und Hilfsstoffe enthalten. Als solche sind insbesondere Flüssigkeiten, die eine Verabreichung über Infiltration bzw. Injektion ermöglichen, wie beispielsweise Wasser oder physiologische Kochsalzlösung, anzusehen.

Die die beschriebenen Komponenten enthaltenden Arzneimittel lassen sich, wie überraschend gefunden wurde, mit großem Erfolg zur Behandlung von Muskelverletzungen verwenden. Die Verwendung erstreckt sich insbesondere auf die Behandlung von Muskelzerrungen, Muskelfaserrissen, Muskelrissen und Muskelkontusionen.

Die erfindungsgemäßen Arzneimittel finden ihre Verwendung darin, daß sie möglichst umgehend nach Eintritt der Verletzung in den traumatisierten Muskelbereich infiltriert werden. Dies ist unabhängig von der Schwere und Art der Muskelverletzung, so daß eine differenzierte und mitunter in den verletzten Muskelbereich eingreifende Diagnostik der Verletzung entfallen kann. Dies ist deswegen von Vorteil, weil damit auch Ärzte mit wenig sportärztlicher Erfahrung die erfindungsgemäßen Arzneimittel gefahrlos und mit sicherem Wirkungseintritt anwenden können.

Die Häufigkeit der Anwendung der erfindungsgemäßen Arzneimittel durch Infiltration schwankt in Abhängigkeit vom objektiven Schweregrad der Verletzung und vom subjektiven Befinden. Bei leichteren Verletzungen bzw. im fortgeschrittenen Stadium der Behandlung kann eine einmalige Anwendung schon zu Schmerzfreiheit und voller Funktionstüchtigkeit der erkrankten Muskelpartien führen. Die Häufigkeit der Applikation kann jedoch auch bis zu ein- oder mehrmaliger täglicher Anwendung bedenkenlos gesteigert werden, wenn die Schwere der Verletzung dies erfordert. Schon die einmalige Behandlung ergibt jedoch meist einen deutlichen Rückgang der Beschwerden. In mehrjährigen klinischen Tests wurden Rezidive an den mit den erfindungsgemäßen Arzneimitteln behandelten Muskelbereichen in keinem Fall beobachtet. Außerdem traten lokale oder systemische Nebenwirkungen in keinem Fall auf.

Ein weiterer Vorteil der Verwendung der erfindungsgemäßen Arzneimittel ist darin zu sehen, daß die leichte örtliche Schmerzreaktion, die kurzzeitig nach der Infiltration auftritt, Aufschluß über die Schwere der Verletzung und die richtige Applikation der Arzneimittel gibt. Dies stellt ein weiteres diagnostisches Hilfsmittel dar, da die Infiltration der erfindungsgemäßen Arzneimittel in gesundes Muskelgewebe keine Schmerzreaktion hervorruft.

Die Erfindung wird durch die nachfolgenden Anwendungsbeispiele näher erläutert.

Beispiel 1

Patient K, 22 Jahre, aktiver Bundesligafußballer; rezidivierende Verletzungen der Oberschenkelbeugemuskulatur rechts mit erheblicher Schmerzhaftigkeit und Funktionseinschränkung und dadurch bedingter Spielunfähigkeit. Die Umfangsmaße der Oberschenkelmuskulatur unterschieden sich nicht meßbar.

In einem Meisterschaftspiel erlitt K nach einem Gewaltschuß plötzlich eine schwere Muskelzerrung des rechten Oberschenkels mit Verdacht auf kleinere Muskelfaserrisse. Eine einmalige Infiltration von 10 ml eines Arzneimittels (Volumenmischung 1 : 1 einer Lösung mit 10 mg/ml Mepivacain-HCl und einer Lösung enthaltend (pro ml) 6 mg Dinatriumdihydrogenadenosintriphosphat, 2 mg Adenosindiphosphorsäure, 2 mg Adenosinmonophosphorsäure, 4 mg Guanosinmonophosphorsäure, 10 mg Adenosin, 2 mg Guanosin, 10 mg Inosin, 2 mg Uridin und 2 mg Chlorkresol) in den schmerzhaften Muskelbezirk ergab schon nach zwei Tagen fast völlige Schmerzfreiheit und volle Funktionstüchtigkeit, so daß bereits nach vier Tagen das Training wiederaufgenommen werden konnte. Zusätzlich erhielt der Sportler die übliche physikalische Behandlung. Seit Abschluß der Behandlung traten keinerlei Beschwerden mehr im Bereich der rechten Oberschenkelmuskulatur auf.

Beispiel 2

Patient H., 32 Jahre, aktiver Bundesligafußballer.

Im Verlauf von fünf bis sechs Jahren konnte dieser Patient wiederholt wegen schmerzhafter Muskelverhärtungen in beiden Oberschenkeln nicht in Spielen eingesetzt werden. Der Patient wurde insgesamt dreimal mit 10 ml des in Beispiel 1 genannten Arzneimittels in jeden der beiden Oberschenkel (Infiltration in · die Oberschenkelbeugemuskulatur) behandelt. Nach viertägiger Trainingspause bestand völlige Schmerzfreiheit. Myogelosen waren palpatorisch nicht mehr erfaßbar. In der Folgezeit traten keine Rezidive in den genannten Muskelgruppen mehr auf.

Beispiel 3

Patient N., 23 Jahre, aktiver Bundesligafußballer.

Im Alter von 19 Jahren wurde in der Oberschenkelbeugemuskulatur des Patienten ein Muskelfaserriß nachgewiesen, der durch mehrwöchige Ruhestellung in Gips sowie physikalische Maßnahmen behandelt wurde. Danach traten, besonders in der kühleren Jahreszeit, häufig Schmerzzustände im Bereich der Muskelnarbe auf, die auch durch eine intensive Spielvorbereitung in Form von Massagen und hyperämisierenden Ölen und Salben nicht zu beherrschen waren.

Der Patient wurde zweimal mit je 7 ml eines Arzneimittels in der tastbaren Muskelnarbe am rechten Oberschenkel im Abstand von 10 Tagen behandelt, das 5 ml einer wässrigen Lösung mit 10 mg/ml Mepivacain . HCl und 2 ml einer wässrigen Nukleinsäurelösung enthielt, die wie in Beispiel 1 zusammengesetzt war. Schon nach der ersten Infiltration trat eine erhebliche Schmerzlinderung ein. Vier Tage nach der zweiten Infiltration kam es zur völligen Schmerzfreiheit und Funktionstüchtigkeit der behandelten Muskelgruppen. Seitdem traten keinerlei Beschwerden des Patienten mehr auf.

Beispiel 4

Patient L., 28 Jahre, aktiver Bundesligafußballer.

Im Verlaufe von drei Jahren wurde bei dem genannten Patienten wiederholt ein Hartspann der linken Wadenmuskulatur diagnostiziert, der zu erheblicher Leistungsminderung, zum Teil sogar zu Spielunfähigkeit führte.

In Abständen von acht Tagen wurde der Patient dreimal mit je 5 ml einer nach Beispiel 1 zusammengesetzten Arzneimittellösung behandelt. Seitdem traten in den genannten Muskelgruppen keinerlei Beschwerden mehr auf.

Beispiel 5

Patient F., 27 Jahre, aktiver Bundesligafußballer.

Bei dem Patienten bestanden drei Wochen lang relativ therapieresistente Muskelverspannungen mit teilweise erheblicher schmerzbedingter Einschränkung der Leistungsfähigkeit, insbesondere beim Kopfballspiel.

In die paracervicale Muskulatur wurden über mehrere Wochen täglich je 5 ml einer nach Beispiel 1 zusammengesetzten Arzneimittellösung infiltriert. Dadurch konnte wieder freie Beweglichkeit der Halswirbelsäule erreicht werden, und ein subjektives Schmerzempfinden wurde nicht mehr angegeben.

Beispiel 6

Patient B., 24 Jahre, aktiver Bundesligafußballer.

Bei dem Patienten wurden mehrere stark - schmerzhafte Myogelosen in der Rückenstreckmuskulatur bei einer geringen S-förmigen Skoliose der BWS diagnostiziert.

Dem Patienten wurden fünf Infiltrationen an fünf aufeinanderfolgenden Tagen, je 2 ml einer nach Beispiel 1 zusammengesetzten wässrigen Arzneimittellösung, in die betroffenen Myogelosen verabreicht. Bei einer Nachuntersuchung nach fünf Tagen konnten keinerlei Muskel härten mehr ertastet werden. Nach der Infiltrationsbehandlung erfolgte keine physikalische Therapie.

Beispiel 7

Patient E., 18 Jahre, Amateurfußballer.

Bei dem Patienten wurde ein frischer Muskelfaserriß im Bereich der rechten Oberschenkelbeugemuskulatur diagnostiziert. In die betroffene Muskelpartie wurden vier Tage nach Eintritt der Verletzung 10 ml der nach Beispiel 1 zusammengesetzten wässrigen Arzneimittellösung infiltriert. Schon nach drei Tagen konnte eine deutliche Schmerzlinderung angegeben werden. Drei Wochen nach der Infiltrationsbehandlung konnte der Patient sein Training ohne volle Belastung wieder aufnehmen. Zur Zeit ist er voll spielfähig und beschwerdefrei.

Beispiel 8

Patient D., 21 Jahre, Amateurfußballer.

Nach einem Spagatschritt trat bei dem Patienten plötzlich heftiger Schmerz in der rechten Leiste auf. Es wurde eine Tendophathie der Adduktorensehenen rechts diagnostiziert.

Im Abstand von drei Tagen wurden zweimal je 2 ml einer nach Beispiel 3 zusammengesetzten wässrigen Arzneimittellösung lokal infiltriert. Nach vier Tagen war der Patient völlig schmerzfrei. Bis heute war kein Rezidiv mehr nachweisbar.

Beispiel 9

Patient B., 18 Jahre, Amateurfußballer.

Während eines Spieles traten bei dem genannten Patienten plötzlich in der rechten Oberschenkelbeugemuskulatur starke Schmerzen auf. Es wurde eine Muskelzerrung diagnostiziert; die entsprechende Muskelpartie war stark druckempfindlich, doch konnte kein sicherer Tastbefund abgegeben werden.

Eine physikalische Therapie an den betroffenen Muskelpartien war ohne Erfolg und brachte keine wesentliche Besserung. Die Oberschenkelbeugemuskulatur wurde durch lokale Infiltration einmal mit 10 ml einer nach Beispiel 1 zusammengesetzten wässrigen Arzneimittellösung behandelt. Nach zwei Tagen stellte sich bei dem Patienten völlige Beschwerdefreiheit ein. Ein Rezidiv war bisher nicht mehr nachweisbar.

Beispiel 10

Patient B., 24 Jahre, Amateurfußballer.

Der Patient klagte über längere Zeit hinweg über Schmerzen in der rechten Leiste nach einem Spreizschritt während eines Fußballspiels. In die rechte Leiste wurden einmal 2 ml einer nach Beispiel 3 zusammengesetzten wässrigen Arzneimittellösung infiltriert. Der Patient konnte fünf Tage nach der Behandlung wieder mit dem Training beginnen und ist bis heute beschwerdefrei.

Neben den angeführten Patienten konnten weitere aktive Sportler mit gleichem Erfolg behandelt werden. Der gleiche Behandlungserfolg stellte sich auch bei stationär bzw. amulant behandelten Patienten aus dem orthopädischen Bereich ein. Auch diese Patienten konnten mit Infiltrationen der erfindungsgemäßen Arzneimittel mit großem Erfolg bei Muskelerkrankungen behandelt werden.

In keinem Fall war eine mehr als dreimalige Infiltrationsbehandlung notwendig. Ein signifikanter Unterschied in der Wirkung zwischen alleiniger Infiltrationsbehandlung und Kombinationsbehandlung mit physikalischer Therapie war nicht festzustellen.

## Ansprüche

1. Arzneimittel, enthaltend in wässriger Phase
(a) 1 bis 100 g/l eines Lokalanästhetikums,
(b) 1 bis 100 g/l eines oder mehrerer Nukleoside aus der Gruppe Adenosin, Guanosin, Inosin, Uridin und deren wasserlöslicher Mono-, Di- und Triphosphatestersalze und
(c) ein oder mehrere mit den Wirkstoffen verträgliche, physiologisch unbedenkliche Konservierungsmittel sowie gegebenenfalls
(d) übliche Träger- und Hilfsstoffe.

2. Arzneimittel nach Anspruch 1, enthaltend in wässriger Phase
(a) 20 bis 30 g/l eines Lokalanästhetikums,
(b) 10 bis 30 g/l eine oder mehrerer Nukleoside aus der Gruppe Adenosin, Guanosin, Inosin, Uridin und deren wasserlöslicher Mono-, Di- und Triphosphatestersalze,
(c) 1 bis 5 g/l eines Konservierungsmittels und
(d) Wasser.

3. Arzneimittel nach Ansprüchen 1 und 2, dadurch gekennzeichnet, daß sie als lokalanästhetisch wirkende Verbindung 1-Methylpiperidin-2-carbonsäure-N-(2',6'-dimethylphenyl-)amid-Hydrochlorid (Mepivacain) enthalten.

4. Arzneimittel nach Ansprüchen 1 und 2, dadurch gekennzeichnet, daß sie als Nukleoside Adenosin, Guanosin, Inosin und Uridin enthalten.

5. Arzneimittel nach Anspruch 4, dadurch gekennzeichnet, daß sie zusätzlich Natriumsalze der Phosphorsäureester von Adenosin, Guanosin, Inosin und Uridin enthalten.

6. Arzneimittel nach Ansprüchen 1 bis 5, dadurch gekennzeichnet, daß sie als Konservierungsmittel Chlorkresol enthalten.

7. Arzneimittel nach Ansprüchen 1 bis 6, enthaltend in wässriger Phase
(a) 5 bis 10 ml einer 5 bis 20 mg/ml 1-Methylpiperidin-2-carbonsäure-N-(2',6'-dimethylphenyl-)amidhydrochlorid (Mepivacain . HCl) enthaltenden wässrigen Lösung;
(b) 5 ml einer wässrigen Lösung, die
30 mg Dinatriumdihydrogenadenosintriphosphat,
10 mg Adenosindiphosphorsäure,
10 mg Adenosinmonophosphorsäure,
20 mg Guanosinmonophosphorsäure,
50 mg Adenosin,
10 mg Guanosin
50 mg Inosin und
10 mg Uridin
enthält sowie
(c) 10 mg Chlorkresol.

8. Verwendung der Arzneimittel nach Ansprüchen 1 bis 7 zur lokalen Infiltrationsbehandlung von Muskelverletzungen.

9. Verwendung der Arzneimittel nach Ansprüchen 1 bis 7 zur lokalen Infiltrationsbehandlung von Muskelzerrungen, Muskelfaserrissen, Muskelrissen und Muskelkontusionen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | ROTE LISTE, 1971, Seite 699, Editio Cantor, Aulendorf/Württ., DE<br>* Seite 699, "LAEVADOSIN", "LAEVADOSIN-2", "LAEVADOSIN-Tabletten-buccal" * | 1-9 | A 61 K  31/70 //<br>(A 61 K  31/70<br>A 61 K  31:445) |
| A | CHEMICAL ABSTRACTS, Band 93, Nr. 3, 21. Juli 1980, Seite 70, Zusammenfassung Nr. 19302s, Columbus, Ohio, US; M.D. BASSON et al.: "Myotoxicity of single and repeated injections of mepivacaine (Carbocaine) in the rat", & ANESTH. ANALG. (CLEVELAND) 1980, 59(4), 275-82<br>* Zusammenfassung * | 1-9 | |
| A | CHEMICAL ABSTRACTS, Band 92, Nr. 21, 26. Mai 1980, Seite 41, Zusammenfassung Nr. 174396y, Columbus, Ohio, US; P.W. BENOIT et al.: "Pharmacologic correlation between local anesthetic-induced myotoxicity and disturbances of intracellular calcium distribution", & TOXICOL. APPL. PHARMACOL. 1980, 52(2), 187-98<br>* Zusammenfassung * | 1-9 | |

RECHERCHIERTE
SACHGEBIETE (Int. Cl.4)

A 61 K

Der vorliegende Recherchenbericht wurde für alle Patentansprüche erstellt

| Recherchenort | Abschlußdatum der Recherche | Prüfer |
|---|---|---|
| DEN HAAG | 04-08-1988 | BRINKMANN C. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes
    Dokument

EPO FORM 1503 03.82 (P0403)